# EUROPEAN PATENT SPECIFICATION

(11) **EP 3 233 782 B1**
(45) Date of publication and mention of the grant of the patent: **02.02.2022**
(21) Application number: 15823054.0
(22) Date of filing: 14.12.2015
(51) Int. Cl.: C07C 51/25, C07C 51/21, B01J 31/04, C09D 191/06

(54) **CATALYTIC OXIDATION OF HYDROCARBONS**
KATALYTISCHE OXIDATION VON KOHLENWASSERSTOFFEN
OXYDATION CATALYTIQUE D'HYDROCARBURES

(30) Priority: 15.12.2014 US 201462091722 P
(43) Date of publication of application: 25.10.2017
(73) Proprietor: The Lubrizol Corporation, Wickliffe, OH 44092-2298 (US)
(72) Inventor: CLARK, Jennifer E., Wickliffe, Ohio 44092-2298 (US); FABER, Ben, Wickliffe, Ohio 44092-2298 (US); MORAN, Gregory E., Wickliffe, Ohio 44092-2298 (US); MINCH, Britt A., Wickliffe, Ohio 44092-2298 (US); BOOTHE, David, Wickliffe, Ohio 44092-2298 (US); HUNT, Daniel W., Hudson, Ohio 44236 (US); KOENIG, Miranda, Belle Plaine, Minnesota 56011 (US)
(74) Representative: D Young & Co LLP
(86) International application number: PCT/US2015/065492
(87) International publication number: WO 2016/100180

(56) References cited:
- GB-A- 849 951
- GB-A- 1 435 675
- US-A- 1 874 322
- US-A- 4 426 229
- US-A- 4 851 043

## Description

### BACKGROUND

The disclosed technology relates to a method for oxidizing an olefin wax, using an organic salt of an alkaline earth metal such as calcium.

Rust preventive coatings have been known to comprise a mineral (hydrocarbon) wax in a solvent or other liquid medium. Such coatings have been applied to metal surfaces, especially ferrous surfaces, as a rust preventative. They may be distinguished from other types of coatings such as paints in that the wax-containing coatings are often of a relatively temporary nature, imparting rust inhibition over a term of days or perhaps years, and in some instances being removed prior to further treatment of or working with the metal surface.

Rust preventative coatings may contain an oxidized wax component, which may contain, among other oxygen containing groups, carboxylic acid groups. The acid content of an oxidized wax is typically reported in terms of acid number (AN), which may be measured according to ASTM D 974. Oxidized waxes may be employed in their original acid form, or they may be reacted with an alcohol such as methanol or butanol to impart methyl or butyl ester functionality in place of the acid.

Oxidation of waxes is commonly performed in the presence of a transition metal catalyst such as manganese or cobalt, in the form of a salt such as the octoate (isooctanoate or 2-ethylhexanoate). Transition metals suitable as oxidation catalysts will typically have multiple available oxidation states such that the transfer of electrons between the metal and the substrate to be oxidized can be seen as the, or a, mechanism involved in the oxidation process.

U.S. Patent 4,426,229, Bolton et al., January 17, 1984, discloses oxidized alpha-olefin waxes. In an example, an oxidized C30+ alpha olefin is substituted in a floor polish formulation for unoxidized C30+ alpha olefin and the resulting film was tough and cohesive. The alpha-olefin may be oxidized in the manner commonly applied to hydrocarbon waxes. Oxidation catalysts such as a wax soluble organic salt such as manganese or cobalt naphthenate are known.

U.S. Publication 2007/0095723, Baralt et al., May 3, 2007, discloses an oxidized olefin wax useful as a pour point depressant in a hydrocarbon composition. The olefin wax may be oxidized in the absence or presence of a catalyst which may comprise manganese or cobalt, among others.

U.S. Patent 2,808,423, Bartlett et al., October 1, 1957, discloses catalytic partial oxidation of hydrocarbons in the presence of a catalyst comprising a manganese salt of a carboxylic acid and an alkaline earth metal salt selected from the group consisting of magnesium salts of carboxylic acid and calcium salts of carboxylic acids.

U.S. Publication 2013/0068134, Yang et al., March 21, 2013, discloses oxidized alphaolefins. Oxidized hydrocarbon waxes may have improved hardness or viscosity. The hardness of waxes is said to be a performance criterion in applications such as polishing, coating, and others. Disclosed is a method comprising contacting an olefin wax with an oxidizing agent and contacting the oxidized olefin wax with an odor reducing material. Oxidation may be in the absence or presence of catalyst which may be manganese or cobalt. The odor-reducing material may comprise a metal compound such as a metal carboxylate, of a Group 1 or Group 2 metal. Among the metals listed are beryllium, magnesium, calcium, strontium, and barium.

U.S. Patent 1,874,322 discloses oxidation of aliphatic hydrocarbons of high molecular weight in the presence of a calcium, barium, strontium, magnesium or aluminium salt of an organic acid.

GB1435675 discloses a process for recovering fatty acids from a mixture of fatty acids with unsaponifiable matter; in particular, working up the mixture of fatty acids and unreacted paraffins obtained by catalytic oxidation of paraffins with gaseous oxygen.

U.S. Patent 4,851,043 discloses water reducible coating compositions comprising an oxidized slack wax, paraffin wax or petrolatum; a low molecular weight polyethylene, polybutene, or polypropylene; naphthenic or paraffinic oil; a salt of a sulfonic acid, a nitrate, a nitrite, a borate or an amine soap of a fatty acid; a mixture of mineral spirits and ethylene glycol monopropyl ether; an unsaturated fatty acid and a fugitive amine; and water.

U.S. Patent 6,512,144, Lewis et al., January 28, 2003, discloses oxidation of hydrocarbons using crystalline manganese (II/III) phosphate compositions, which may have a composition expressed by an empirical formula (A^{*a*+})*ᵥ*(Mn^{*b*+})(M^{*c*+})*ₓ*P*_{y}*O*_{z}* where A is a structure directing agent including (among others) "alkaline earth metals (except calcium)."

While transition metals such as Mn are satisfactory oxidation catalysts, in certain oxidation systems they can lead to excessive degradation or crosslinking of the wax with undesirable changes in viscosity. It may also be desirable to prepare oxidized waxes which do not contain residual transition metal catalyst or from which such catalyst does not need to be removed.

### SUMMARY

The disclosed technology provides a method for oxidizing an olefin wax of 10 to 100 carbon atoms, comprising: (a) mixing said olefin wax with a salt of an alkaline earth metal in an amount to provide 0.001 to 0.03 weight percent of the alkaline earth metal of the reaction mixture, the anion of said salt comprising 4 to 36 carbon atoms; (b) heating said mixture to 100 to 180 °C, or 140-160 °C; and (c) supplying to said heated mixture a molecular oxygen-containing gas; wherein the method is conducted in the substantial absence of manganese or cobalt compounds, such that the amount of manganese or cobalt is less than 1 % or less than 0.1% or less than 0.01 percent by weight of the amount of the alkaline earth metal from the alkaline earth metal salt.

### DETAILED DESCRIPTION

The disclosed technology is particularly useful for oxidizing certain waxes, in particular, olefin waxes including α-olefin waxes (sometimes simply referred to as olefins or α-olefins). An olefin wax may comprise olefins or α-olefins having 16 to 40 carbon atoms or, in other embodiments, 16-36 or 16 to 26 or 20-24 or 24-28 or 26-28 carbon atoms, or in some instances 30 carbon atoms and greater, e.g., 30-48, 30-36. Olefins and alpha olefins having such carbon numbers are commercially available materials which may be prepared, for instance, by stepwise addition of ethylene to certain organometallic compounds (e.g., triethylaluminum) in the presence of a catalyst such as colloidal nickel. The term "α-olefin" refers to an olefin that has a double bond between the first and second carbon atom of the longest contiguous chain of carbon atoms. The olefins may be linear or branched. The terms olefin or α-olefin do not exclude the possible presence of one or more additional double bonds at other positions in the carbon chain, nor does it exclude the possible presence of heteroatoms within the carbon chain or pendent from the chain, such as oxygen atoms. Commercial α-olefins are typically mixtures of individual molecules having the number of carbon atoms specified in the above ranges and optionally including relatively small amounts of molecules outside those ranges. In one embodiment, the α-olefin is a mixture of hydrocarbon molecules.

Further properties that can be utilized to describe the olefin wax from which the oxidized olefin or α-olefin wax may be prepared include the olefin content of the wax, the carbon number composition, average molecular weight, the types of olefins present in the olefin wax (alpha olefin, linear alpha olefin, vinylidene olefin, internal olefin, and linear internal olefin), and the content of the olefin types present within the α-olefin wax.

In one embodiment, olefin wax or α-olefin wax may comprise greater than 75 mole percent olefins; alternatively, greater than 85 mole percent olefins; alternatively, greater than 90 or 95 mole percent olefins. In other embodiments, the α-olefin wax may consist essentially of olefins. The olefin wax can contain some saturated paraffin wax and still be useful in the context of the disclosed technology. In some embodiments, the α-olefin wax contains less than 25 mole percent saturated paraffin wax; alternatively, less than 15 or 10 or 5 mole percent saturated paraffin wax.

In certain embodiments, the olefin or α-olefin wax may have a proportion of waxes within a defined carbon number range. In some embodiments, the olefin wax can comprise greater than 70 weight percent olefins having from 20 to 24 carbon atoms; alternatively, greater than 80 or 85 or 90 or 95 weight percent olefins having from 20 to 24 carbon atoms. In other embodiments, the olefin wax can comprise greater than 50 weight percent olefins having from 24 to 28 carbon atoms; alternatively, greater than 60 or 70 or 80 or 90 weight percent olefins having from 24 to 28 carbon atoms. In yet other embodiments, the olefin wax can comprise greater than 50 weight percent olefins having from 26 to 28 carbon atoms; alternatively, greater than 60 or 70 or 80 or 90 weight percent olefins having from 26 to 28 carbon atoms. In yet further embodiments, the olefin wax can comprise greater than 70 or greater than 8 or 85 or 90 or 95 weight percent olefins having at least 30 carbon atoms. In one embodiment, the olefin wax comprises an α-olefin of 16 to 35 or to 26 carbon atoms.

Alternatively, the olefin or α-olefin wax may be described in terms of its average molecular weight, especially number average molecular weight. In an embodiment, the olefin wax has an average olefin molecular weight greater than 260 grams/mole. In some embodiments, the olefin wax has an average olefin molecular weight greater than 330 or greater than 400 grams/mole. In an embodiment, the olefin or α-olefin wax may have has an average molecular weight ranging from 260 grams/mole to 1000 grams/mole, or 260 to 750 or 260 to 550 grams/mole. In some embodiments, the molecular weight may be 260-340, or 280 to 320, or 290 to 310, or 330-420, or 350-400, or 360-390, or 440-550, or 560-530, or 480-510 grams/mole.

In some embodiments, the olefin or α-olefin wax can comprise greater than 30 mole percent alpha olefins, or greater than 45 or 50 or 75 or 90 or 95 mole percent alpha olefins. In some embodiments, the α-olefin wax can comprise 50 to 99 mole percent, or 55 to 98, or 60 to 97, or 65 to 95 mole percent alpha olefins. In certain embodiments, the wax can comprise linear alpha olefins, such as greater than 30 mole percent, or greater than 45 or 60 or 75 mole percent linear alpha olefins, such as 30 to 99 or 40 to 95 or 59 to 90 mole percent linear alpha olefins. A more detailed description of α-olefins may be found in U.S. Publication 2007/095723, paragraphs 0035 to 0045.

The wax is oxidized by heating in the presence of air or other molecular-oxygen-containing gas in the presence of a catalytic amount of an alkaline earth metal salt. The alkaline earth metals include those in Group II of the periodic table of the elements, and especially magnesium, calcium, strontium, and barium, and in some embodiments especially calcium or barium; or especially calcium.

The catalytic amount of the alkaline earth metal salt is an amount suitable to provide 0.001 to 0.03 weight percent of the alkaline earth metal of the reaction mixture, such as 0.001 to 0.03 weight percent of calcium. Other suitable amounts of the alkaline earth metal in general or of calcium in particular may be 0.005 to 0.027 weight percent or 0.008 to 0.025 weight percent or 0.010 to 0.021 weight percent. In certain embodiments the amount of the salt of the alkaline earth metal may be 0.01 to 0.25 weight percent, or about 0.08 weight percent, of the amount of the olefin wax.

The alkaline earth metal salt will have a suitable anion to provide a measure of solubility or miscibility of the salt in the reaction mixture. Since the reaction mixture may consist essentially of the olefin wax, solubility, miscibility, or otherwise compatibility with such wax under oxidation conditions will be desirable. In certain embodiments the anion may be a carboxylate. In the present invention, the anion of the salt of the alkaline earth metal comprises 4 to 36 carbon atoms. In other embodiments, the anion of said salt may comprise 6 to 18 or 6 to 12 or 8 to 12 or 8 carbon atoms. Suitable materials include calcium octoate, also referred to as calcium 2-ethylhexanoate, as well as calcium cyclohexanebutyrate, calcium stearate, calcium oleate, calcium hexanoate, calcium neodecanoate, calcium naphthenate (a mixture of anions), and barium 2-ethylhexanoate. In one embodiment, the salt of the alkaline earth metal comprises calcium di-2-ethylhexanoate (i.e., dioctoate). Many of such materials are commercially available and may be prepared by the neutralization of the corresponding carboxylic acid of a calcium base such as calcium oxide or calcium hydroxide. Other salts than carboxylates may include alkoxides such as calcium butoxide or calcium 2-ethylhexoxide. The alkaline earth metal salt may be added to the oxidation mixture in the indicated salt form, or the salt may be formed *in situ* from reaction of an alkaline earth metal base with an appropriate carboxylic acid or alcohol. In certain embodiments the only metal present will be the alkaline earth metal, such as calcium, except for trace amount or contaminant amounts of other metals that do not catalytically affect the oxidation reaction (the metal may thus consist essentially of the alkaline earth metal). Other such metals may include alkali metals such as sodium, lithium, or potassium, or iron.

Other metals, such as manganese or cobalt, have been used in the past as catalysts for oxidation of hydrocarbons. The method of the present invention is conducted in the substantial absence of manganese or cobalt compounds, such that the amount of manganese or cobalt is less than 1% or less than 0.1% or less than 0.01% by weight of the amount of the alkaline earth metal from the alkaline earth metal salt. Acceptable amounts of Mn or Co may thus be less than 0.0003 percent by weight (3 ppm) or less than 1 ppm or less than 0.5 ppm or 0.1 ppm of the oxidation mixture.

While the oxidation of the olefin wax may be conducted using the neat wax, without any solvent or diluent, it is also possible to include a solvent or diluent. Such a material may be a hydrocarbon solvent which may be or comprise an oil. It should be recognized, however, that any solvent may itself be subject to oxidation, which may or may not be desirable. If desired, any solvent or oxidized solvent may be removed from the oxidized wax (e.g., the oxidized olefin wax) after reaction, by evaporation, distillation, or other known conventional means. In one embodiment, no solvent or diluent will be used beyond the oil that may be inherently present in the olefin wax. In certain embodiments, moreover, the alkaline earth metal catalyst may itself be provided along with a solvent or diluent such as a hydrocarbon or a hydrocarbon oil. The amount of such diluent will typically be relatively small in comparison with the amount of the wax to be oxidized, such as 1 to 4%, and this amount of diluent may be retained in the oxidate, if desired.

The oxidation reaction of the present invention is conducted at an elevated temperature of 100 °C to 180 °C, alternatively 120 °C to 170 °C or 140 °C to 160 °C.

The heated mixture of wax and alkaline earth metal catalyst may be treated with a stream of a molecular oxygen-containing gas under pressure, typically with stirring or other mixing. The reaction may take place in a batch reaction vessel or in other variations, such as a stirred continuous reaction process or in a continuous process as in an extruder or other plug flow device. The molecular oxygen containing gas may conveniently be air, although mixtures of air enhanced or depleted in oxygen may also be used. (Use of gas with an excess of molecular oxygen may lead to accelerated reaction and should be conducted, if at all, with caution and adequate safeguards to avoid excessively vigorous reaction.)

The molecular oxygen-containing gas may be supplied at near normal atmospheric pressure or it may be supplied at elevated pressure. Typical pressures may be 450 to 1800 kPa (50 to 250 psi gauge or 65 to 265 psi absolute), or alternatively 700 to 1500 kPa or 750 to 1400 kPa.

The oxidation reaction will be continued until the desired degree of oxidation of obtained. The extent of oxidation may be determined as Acid Number, ASTM D974, using samples obtained as described in greater detail in the Examples section. The required reaction time may be, in various embodiments, 100 to 1000 minutes, or 115 to 800 minutes, or 200 to 600 minutes, or 250 to 550 minutes, or approximately 300 minutes.

After the oxidation of the wax, the alkaline earth metal catalyst may optionally be retained in the oxidized wax, as its presence is not deleterious for many applications. Alternatively, if desired, some or all of the alkaline earth metal may be removed from the oxidized wax. This may be by a heated water wash, e.g., agitating with water above the melting point of the oxidized wax, followed by removal of the water.

The resulting oxidized wax may be esterified if desired. Esterification may be by reaction of the oxidized wax with an alcohol, optionally in the presence of a catalytic amount of an acid such as sulfuric acid. The alcohols may contain 1 to 28 carbon atoms, or 1 to 8, or 1 to 4, or 1, 2, 3, or 4 carbon atoms, or mixtures thereof. The alcohols may be linear or branched, that is, in any of their isomeric forms, e.g., methanol, ethanol, propyl alcohol, isopropyl alcohol, n-butyl alcohol, sec-butyl alcohol, isobutyl alcohol, t-butyl alcohol, 2-ethylhexylanol, 2-butyl octanol, 2-hexyl decanol, 2-octyl dodecaonol, 2-decyl-tetradecanol, 2-dodecyl-hexadecanol, or mixtures thereof. If there is more than one carboxylic acid group on a particular oxidized olefin wax moiety, one or more of the carboxylic groups may be esterified and one or more may remain unesterified; moreover, the esterifying alcohol may be the same or different for each of the multiple carboxylic groups. In one embodiment, the oxidized olefin wax may be esterified with methanol. In one embodiment, the oxidized olefin wax may be esterified with n-butanol or with iso-butanol or a mixture of butanols.

The esterification of the oxidized wax will consume some but not necessarily all of the acidic functionality originally imparted by the oxidation process. The extent of reaction may be evaluated by measuring the acid number before and after the esterification reaction. For example, if an olefin wax is oxidized to an acid number of 60 to 80, after its esterification the acid number may be 10 to 30 or 12 to 28 or 15 to 25 or 17 to 25, or less than 20. In certain embodiments esterification with methanol may lead to an acid number of 17 to 25. In certain embodiments esterification with butanol may lead to an acid number less than 20.

The oxidized wax as disclosed herein may be used to prepare a coating composition. The amount of the oxidized olefin wax in a composition may be 10 to 45 percent by weight of the composition, or 15 to 40, or 20 to 40, or 25 to 38 percent by weight.

As well as an oxidized olefin wax, the disclosed composition may also contain unoxidized wax of various types, such as unoxidized α-olefin or other hydrocarbon wax such as paraffin wax. The unoxidized wax, of present, may be present in an amount of up to 40 weight percent of the coating composition, such as 10 to 40 or 15 to 35 weight percent.

The composition may also contain one or more hydrocarbon-soluble ester-containing polymers, which may function as solubility agents. A solubility agent is useful to assist in assuring solubility of the various components such as the oxidized olefin wax in the coating composition, both within a concentrate and in a subsequent dilution in oil or solvent. Solubility agents may also be or comprise pour point depressants.

The solubility agent may be a hydrocarbon-soluble ester-containing polymer having at least one branch or side chain of 10 to 36 carbon atoms and an overall number average molecular weight of 5000 to 300,000. In one embodiment the ester functionality of the polymer will be pendant from the main polymer chain and the branches or side chains will be associated with the pendant ester functionality. This would be in distinction from polymers in which the ester functionality is within the main polymer chain, such as, for instance, polyethylene terephthalate. The ester-containing polymer may comprise a copolymer of an alkyl ester with vinyl acetate or with a vinyl aromatic compound, wherein the alkyl group contains 12 to 22 carbon atoms. More particularly, in certain embodiments the ester-containing polymer may comprises units of polymerized ester of maleic acid or fumaric acid.

In certain embodiments, the ester-containing polymer may be a polymer comprising at least one monomer of a least one alkyl ester of an ethylenically unsaturated 1,2-diacid, wherein the alkyl groups of the ester contain on average 10 to 36 carbon atoms. This material is a polymer which has a substantially carbon chain backbone derivable from the addition polymerization of an ethylenically unsaturated diacid, optionally with other comonomers, described below. The polymerized acid groups are at least partly, and may be substantially completely, in the form of alkyl esters. Diacids which are capable of polymerization may encompass those ethylenically unsaturated acids having 3 to 6 carbon atoms, including those with α,β-ethylenic unsaturation. Specific materials include fumaric acid, maleic acid, itaconic acid, and citraconic acid and their reactive equivalents.

The polymers useful in the present technology may be prepared directly from an ester of the acid, from the acid itself, or (in the case of certain diacids) the anhydride, or from other reactive monomers. If the polymer is prepared from one of the materials other than the ester it can be converted into the ester form by reaction of the polymer with a suitable alcohol or by other well-known reactions.

The alcohol with which the acid monomer or the polymeric acid functionality or equivalent thereof is reacted to form the ester is an alcohol with an alkyl chain containing 10 to 36 carbon atoms, such as 10 to 28 or 12 to 22 carbon atoms. The alkyl group need not be derived from a single alcohol of a single chain length, however, but can be derived from a mixture of alcohols if desired, provided that at least on average the chain lengths of the alcohol portion fall within the desired range.

The ester-containing polymer may also contain other monomers derived from ethylenically unsaturated compounds. These comonomers can be short chain ester-containing monomers such as vinyl alkanoates where the alkanoate moiety contains up to 8 carbon atoms or up to 4 carbon atoms, such as vinyl acetate, vinyl propionate, and vinyl butyrate. Alternatively, or additionally, the polymer can contain short chain alkyl ether comonomers, where the alkyl group has up to 8 carbon atoms e.g., ethyl vinyl ether, propyl vinyl ether, and the butyl vinyl ethers.

Other possible monomers include nitrogen-containing monomers, or vinyl acetate monomers. Other available copolymerizable monomers may include □-olefins, including ethylene or propylene, or vinyl aromatic monomers such as styrene, or carbon monoxide or sulfur dioxide. The amount of supplemental comonomers, if any, may be sufficiently low that the polymer substantially retains its character as a hydrocarbyl alkenoate polymer, modified by the presence of the above-defined comonomers. (As used herein, "hydrocarbyl" is used in its ordinary sense, which is well-known to those skilled in the art. Specifically, it refers to a group having a carbon atom directly attached to the remainder of the molecule and having predominantly hydrocarbon character. Examples of hydrocarbyl groups include hydrocarbon substituents, including aliphatic, alicyclic, and aromatic substituents; substituted hydrocarbon substituents, i.e., substituents containing non-hydrocarbon groups which, in the context of this technology, do not alter the predominantly hydrocarbon nature of the substituent; and hetero substituents, that is, substituents which similarly have a predominantly hydrocarbon character but contain other than carbon in a ring or chain. A more detailed definition of the term "hydrocarbyl substituent" or "hydrocarbyl group" is found in paragraphs [0137] to [0141] of US Publication 2010-0197536.)

The ester-containing polymers of can be prepared by known methods. In one example, a di-(C₁₂-C₁₄) fumarate is mixed with an appropriate amount of vinyl acetate or styrene, and the reactants mixed and heated, with or without a solvent or diluent, in the presence of a small amount of an initiator at 25°C to 150°C, or up to 100°C. The polymerization can be run continuously or batchwise. The polymer as used in the disclosed technology may have a number average molecular weight of 5,000 to 400,000, or 6,000 to 100,000, or 8,000 to 50,000, or 10,000 to 45,000. Details of such polymerizations are well known to those skilled in the art and are reported in greater detail in U.S. Patent 3,250,715. The polymers are also described in U.S. Patent 6,017,370.

Examples of suitable ester-containing monomers may those as summarized in the following Table:

| Polymer type | Pendant chain length, C atoms | Pendant chain type | Mₙ |
|---|---|---|---|
| α-olefin/maleic anhydride | 8, or 8-10, or 12, or 12-14, or 12-18, or 16, 22, or 24-28, or mixtures thereof | linear or branched or mixtures thereof | 15,300 to 85,200, or 25,000 to 30,000 |
| styrene/maleic anhydride | 4, or 8, or 8-10, or 12, or 12-18, or 14-15, or 16, or 16-18, | linear or branched or mixtures thereof | 30,000 to 300,000, or |
| | or 18-22, or 20-24; optionally including a small amount of n-aminopropylmorpholine | | 40,000 to 160,000, or 50,000 to 100,000 |
| polymethacrylate | 12 & 18 (varying ratios) | linear | 32,100 to 87,400 |
| vinyl acetate/ fumaric acid | 12-22, 18-22, or 20+ | linear | 50,000 to 300,000 |

The ester-containing polymer (solubility agent) may be present in an amount of 0.1 to 10 percent by weight, or 0.5 to 5 or 0.7 to 2 percent by weight. The relative weight ratio of oxidized olefin wax component to the ester-containing polymer may be 400:1 to 4:1 or 200:1 to 10:1 or 100:1 to 20:1 or 50:1 to 30:1.

The coating compositions of the disclosed technology may also include a carboxylic acid having at least 10 carbon atoms. The acid may be a monoacid or it may be a diacid or polyacid. By "polyacid" is meant a material having three or more carboxylic acid groups. If the diacid or polyacid is a diacid having two carboxylic acid groups on adjacent carbon atoms, then the diacid may have at least 14 total carbon atoms. In one embodiment, the diacid or polyacid is a diacid having at least 16 carbon atoms, the two carboxylic acid groups being separated by at least 6 carbon atoms. In such an embodiment, there may also be more than two carboxylic acid groups present, but if so, then at least two of them may be separated by at least 6 carbon atoms which are not themselves substituted by a carboxylic group. The separating carbon atoms in such an embodiment are typically non-aromatic and, in one embodiment, they comprise a carbon chain, that is, without interruption by inserted oxygen or nitrogen atoms. In certain embodiments the carboxylic groups may be separated by 8 to 24 carbon atoms, or 10 to 20, or 12 to 20, or 14 to 18 carbon atoms.

One type of diacid is known as dimer acids or dimerized acids. Dimer acids are products typically prepared by dimerization of long chain, e.g., C18, unsaturated fatty acids. They are often prepared by self-condensation of oleic acid or tall oil fatty acids. Dimer acids are mixtures of relatively high molecular weight materials, m.w. around 560, yet are liquid at room temperature. They are commercially available materials that may be prepared by either a Diels-Alder reaction or by a free radical route, or by catalysis on a substrate such as clay. In another embodiment, a diacid may include a hydrocarbyl-substituted succinic acid having at least 14 carbon atoms including the four carbon atoms of the succinic acid moiety, e.g., succinic acid substituted with a 10-carbon alkyl. In other embodiments there will be at least 12, 14, 16, or 18 carbon atoms in such an alkyl substituent (for a total number of 16, 18, 20, or 22 carbon atoms). The number of atoms in the alkyl substituent may be up to 36 or 30 or 24 or 22 carbon atoms. In another embodiment, the diacid may be an α,ω-alkylene diacid, of at least 10 or 12 carbon atoms, and up to, for instance, 36 or 24 or 18 carbon atoms. Examples include 1,10-decanedioic acid, 1,12-dodecanedioic acid, and 1,18-octadecanedioic acid.

Alternatively, the acid may be a monocarboxylic acid, having at least 10 carbon atoms. In some embodiments it may have a carbon chain of 8 to 24 carbon atoms. Such acids are often derived by hydrolysis of natural oils or fats. They may be saturated or unsaturated and may contain additional substituents such as a hydroxy group. These acids, sometimes referred to as fatty acids, are well known and may typically include stearic acid or hydroxystearic acid.

The amount of the above-described carboxylic acid, whether monoacid, diacid, or polyacid in the coating composition of the disclosed technology, may be 0.3 to 10 percent by weight, or 0.5 to 8, or 1 to 5 percent by weight, calculated excluding the presence of any volatile diluent.

The formulation may also contain a metal salt of an alkylarylsulfonate having one or more hydrocarbyl or alkyl groups of sufficient length to provide solubility in a hydrocarbon oil, e.g., having at least 12 carbon atoms and up to 200 carbon atoms, such as 18 to 100 or 24 to 48 carbon atoms in the combined alkyl or hydrocarbyl groups or, alternatively, in the longest of such groups if there is more than one. Examples of metal sulfonate salts include relatively low molecular weight salts such as calcium mono-, di-, or tri-nonyl naphthalene sulfonate (or mixtures of mono-, di-, and tri-alkyl species) and relatively higher molecular weight salts such as calcium oligo- or poly-propene benzenesulfonates or -toluenesulfonates. These may be neutral salts or overbased salts. Neutral salts are those that contain approximately or exactly a stoichiometric amount of metal ion to neutralize the acid functionality of the alkarylsulfonic acid. Overbased salts are prepared by reaction with a stoichiometric excess of metal, such as calcium, barium, magnesium, potassium, zinc, or sodium, in the form of a basic compound such as, in the case of calcium, the oxide, hydroxide or, ultimately, the carbonate as a result of treatment with carbon dioxide. Overbased materials are well known in the lubricant industry as overbased detergents and may also function as surfactants or wetting agents. The salt may be a calcium salt. Overbased detergents are described in detail in US Patents 2,501,731; 2,616,905; 2,616,911; 2,616,925; 2,777,874; 3,256,186; 3,384,585; 3,365,396; 3,320,162; 3,318,809; 3,488,284; and 3,629,109. The amount of the metal salt of the alkylarylsulfonate in the disclosed coating composition may be 2 to 30 percent by weight, or 3 to 30, or 3 to 25, or 4 to 20, or 5 to 15 percent by weight, on an oil-free basis.

The coating composition may also contain an oil in an amount sufficient to dissolve the metal salt of the alkylarylsulfonic acid. The oil may be a natural or synthetic oil, an oil derived from hydrocracking, hydrogenation, and hydrofinishing, an unrefined, refined, re-refined oil, or mixtures thereof. A more detailed description of unrefined, refined and re-refined oils is provided in International Publication WO2008/147704, paragraphs [0054] to [0056] and in the corresponding paragraphs of US-2010-0197536. A more detailed description of natural and synthetic lubricating oils is described in paragraphs [0058] to [0059] respectively of WO2008/147704. Synthetic oils may also be produced by Fischer-Tropsch reactions and typically may be hydroisomerized Fischer-Tropsch hydrocarbons or waxes. In one embodiment oils may be prepared by a Fischer-Tropsch gas-to-liquid synthetic procedure as well as other gas-to-liquid oils. In one embodiment, the oil may be selected from any of the base oils in Groups I-V as specified in the American Petroleum Institute (API) Base Oil Interchangeability Guidelines. The five base oil groups are as follows: Group I: >0.03% sulfur and/or <90% saturates and viscosity index (VI) 80 to 120; Group II: ≤0.03% sulfur and ≥90% saturates and VI 80 to 120; Group III: <0.03% sulfur and ≥90% saturates and VI >120; Group IV: all polyalphaolefins; Group V: all others. Groups I, II and III are mineral oil base stocks. Natural oils also include vegetable oils such as coconut oil, castor oil, olive oil, peanut oil, rapeseed (canola) oil, corn oil, sesame seed oil, cottonseed oil, soybean oil, palm oil, sunflower oil, safflower oil, linseed oil, and tung oil. In one embodiment the oil is a hydrocarbon oil. The amount of oil in the coating composition may be 2 to 80 percent by weight, 5 to 70 or 10 to 45 or 15 to 35 percent by weight.

The coating composition may also, optionally, contain one or more additional components or additives that are conventionally used in coating metals, in conventional amounts. Such optional additives may include antioxidants, polymeric film formers such as acrylic polymers, surfactants (including wetting agents), colorants, defoamers, demulsifiers, and rheology modifiers.

The coating composition may also optionally contain a volatile diluent. By "volatile diluent" is meant a normally liquid component that has a volatility greater than that of an oil such as mineral oil. The volatile diluent may comprise water or one or more organic solvents. The diluent may thus comprise a volatile organic solvent such as naphtha (also known as petroleum ether), mineral spirits, kerosene, or ethyl lactate. Among these materials may be hydrocarbon solvents. Such materials may have a boiling point of 30 to 60 °C or higher temperatures, up to a range of 175 to 280 °C. Some such volatile diluents may have a boiling range of 130-210 °C; others 196-205 °C. Overall, a diluent may be considered volatile if its boiling point is less than 280 °C. The volatile diluent may be present in a concentrate of the foregoing components, if desired, although most commonly the diluent, or the majority of the diluent will be added in preparing the fully formulated, diluted coating composition. The amount of diluent will typically be an amount to provide for appropriate viscosity and rheological performance so that the coating composition may be applied to a substrate such as a metallic article or surface. Thus, if the concentrate is diluted to 20 percent in the final coating composition, the total amount of diluent will typically 80 percent additional solvent or diluent to make the dilution (in addition to the oil dissolving the metal salt, which is not counted toward the amount of the volatile diluent). The overall total amount of the diluent (if present) will depend, of course, on the amount of dilution used to prepare the final coating composition and so may be 40 to 98 percent by weight, or 60 to 98, or 40 to 95, or 60 to 88, or 80 to 86, or 82 to 84 percent by weight. The volatile diluent may be removed from the composition by evaporation, after it is used to coat a surface as described below. The amount of the other components will typically be 100% by weight less the amount of the optional volatile diluent, such as 2 to 60 weight percent and other amounts that may be readily determined by the skilled person.

Coating formulations containing waxes such as oxidized α-olefin waxes and other waxes are described in greater detail in U.S. Provisional Application 61/918166, filed December 19, 2013.

The coating composition may be especially useful for coating metal surfaces including as ferrous metal surfaces that are subject to rust, such as steel and cast iron, although many non-ferrous metal surfaces can derive protection and thereby benefit from the described compositions as well. Some surfaces that may benefit from the coating described herein include aluminum surfaces, galvanized iron phosphate surfaces, and zinc phosphate surfaces. In some embodiments, the coatings may be useful to provide protection to an article for a period of weeks or months, for instance, during shipping, storage, or manufacture, rather than for years as might be typical of a paint or other permanent coating. In other embodiments, however, the coating may be left on the surface indefinitely (e.g., even for a period of years). Accordingly, the present invention also provides an article coated with the oxidized olefin wax or coating composition disclosed herein.

The disclosed technology may be used to provide protection, such as rust protection, to a surface, comprising applying thereto the above-described coating composition. The coating composition of the disclosed technology may be applied to an article or surface by brushing, rolling, dipping, curtain coating, doctor blading, wiping, or spraying. Spraying may be effected using an airless spray/pump device, conventional air spray, or an electrostatic method. The applied coating may have any thickness desired, such as 2 µm to 200 µm or 5 to 150 µm, or 10 to 100 µm.

The amount of each chemical component described is presented exclusive of any solvent or diluent oil, which may be customarily present in the commercial material, that is, on an active chemical basis, unless otherwise indicated. However, unless otherwise indicated, each chemical or composition referred to herein should be interpreted as being a commercial grade material which may contain the isomers, by-products, derivatives, and other such materials which are normally understood to be present in the commercial grade.

Various preferred features and embodiments will be described below by way of non-limiting illustration.

### EXAMPLES

Ex. 1 through 5. A batch of α-olefin wax (typically about 360 g unless otherwise indicated, initial kinematic viscosity at 100 °C being about 3 mm²/s (cSt)) is charged to a stirred stainless-steel reactor along with a catalyst, as shown in Table I. The reactor is sealed and is heated to 160 °C. When the reactor reaches temperature, a flow of compressed air through the mixture is begun at 0.11 standard cubic meters (4 standard cubic feet) per hour, at a pressure of 790 kPa (100 psig, 115 psia). At 30 to 60 minute intervals the vessel is temporarily depressurized and a sample of the reaction mixture is removed through a port. The sample is analyzed for Acid Number by ASTM D974, and the oxidation rate, in terms of Acid Number/minute is calculated. The overall oxidation rate is reported in Table I, being an average rate over the course of the reaction, the rate of oxidation being reasonably constant throughout the reaction time. (Since the specific rate may depend on experimental details such as air flow or stirring, direct comparisons should not be made between oxidations conducted in different equipment or under significantly different conditions.) When the desired Acid Number is reached (55-70 for these examples), the reactor is cooled and the oxidate is washed with deionized water and allowed to separate in an 80 °C oven for 1 hour. The aqueous layer is removed and the oxidate portion is heated, with stirring, at 110 °C until it appears to be dry. At this point, a final Acid Number measurement is made. Results are shown in Table I:

**Table I**

| Example: | Ex. 1 | Ex. 2 | Ref. Ex. 3 | Ref. Ex. 4 | Ex. 5^{∗} |
|---|---|---|---|---|---|
| α-Olefin | 98.3 | 96.6 | 100 | 99.3 | 98.3 |
| Ca octoate (5% chemical in 95% oil diluent | 1.68 | 3.36 | - | - | 1.68 |
| Mn octoate (12% chemical in 88% oil diluent) | - | - | - | 0.7 | - |
| Reaction Rate (AN/min) | 0.188 | 0.252 | 0.168 | 0.167 | 0.17 |
| Final Acid Number | 64.0 | 61.4 | 68.3 | 60.5 | 65.9 |
| Kinematic Viscosity of product, 100 °C , mm²/s (cSt) | 13.7 | 11.7 | 13.0 | 53.8 | 23.8 |

| | | | | | |
|---|---|---|---|---|---|
| ^{∗}Run on a larger scale (pilot plant); rate is not directly comparable to the smaller scale. | | | | | |

The results indicate that the oxidation in the presence of the calcium octoate proceeded more rapidly than in the presence of manganese octoate or in the absence of any catalyst. The results also show less undesirable increase in viscosity for the Ca catalyzed examples than for the Mn catalyzed reference example.

Ex. 6 through 14 illustrate the oxidation of either α-olefin (covered by the present invention) or slack wax (not covered by the present invention) using a Ca catalyst, under conditions similar to those of examples 1-5 except at an air pressure of 1400 kPa (190 psig, 205 psia). Reaction is continued to a target AN of 77 to 83. Results are shown in Table II.

**Table II**

| Example: | Ref 6 † | Ref 7 | Ex 8 | Ex 9 | ReflO | Ref11 ^{∗}# | Ref12 * | Ex 13 ^{∗}# | Ex 14 * |
|---|---|---|---|---|---|---|---|---|---|
| α-Olefin | 99.3 | 99.3 | 98.3 | 96.6 | | | | | |
| Slack Wax | | | | | 99.3 | 99.3 | 99.3 | 98.3 | 98.3 |
| Ca octoate (5% chemical in oil diluent) | | | 1.68 | 3.36 | | | | 1.68 | 1.68 |
| Mn octoate (12% chemical in oil diluent) | 0.7 | 0.7 | | | 0.7 | 0.7 | 0.7 | | |
| Reaction Rate (AN/min) | 0.107 | 0.14 | 0.211 | 0.321 | 0.33 | 0.117 | 0.178 | 0.155 | 0.216 |
| Final Acid Number | 74 | 76.3 | 78.8 | 81.1 | 90.3 | 82.6 | 79.9 | 81.9 | 84.7 |
| Kinematic Viscosity of product, 100 °C mm²/s | 93.5 | 47.2 | 26.7 | 7.35 | 6.51 | 9.88 | 7.89 | 6.78 | 5.90 |

| | | | | | | | | | |
|---|---|---|---|---|---|---|---|---|---|
| ^{∗}= run on a larger scale (pilot plant); rate not directly comparable † = two runs, combined, values averaged # = run using a different (old) air delivery system, so the reaction rate for these two examples is reduced | | | | | | | | | |

Ex. 15 through 27 illustrate the oxidation of slack wax (not covered by the present invention) or α-olefin wax using a Ca catalyst, under conditions similar to those of examples 1-5, but continuing the oxidation to an target AN of 24 to 40. Results are shown in Tables III and IV.

**Table III**

| Example: | Ref 15 | Ref 16 | Ref 17 | Ref 18 | Ex.19 | Ex.20 |
|---|---|---|---|---|---|---|
| Slack Wax | 99.3 | 99.3 | 100 | 100 | 98.3 | 98.3 |
| Ca octoate (5% chemical in oil diluent | | | | | 1.68 | 1.68 |
| Mn octoate (12% chemical in oil diluent) | 0.7 | 0.7 | | | | |
| Reaction Rate (AN/min) | 0.273 | 0.363 | 0.140 | 0.124 | 0.336 | 0.337 |
| Final Acid Number | 32.8 | 30.5 | 34.0 | 29.7 | 32.8 | 33.3 |
| Kinematic Viscosity of product, 100 °C , mm²/s | 5.19 | 4.77 | 4.58 | 4.42 | 4.42 | 4.55 |

**Table IV**

| Example: | Ref21 | Ref22 | Ref23 | Ref24 | Ex 25 | Ex 26 | Ex27^{∗} |
|---|---|---|---|---|---|---|---|
| α-Olefin | 100 | 100 | 99.3 | 99.3 | 98.3 | 98.3 | 98.3 |
| Ca octoate (5% chemical in oil diluent | | | | | 1.68 | 1.68 | 0.68 |
| Mn octoate (12% chemical in oil diluent) | | | 0.7 | 0.7 | | | |
| Reaction Rate (AN/min) | 0.282 | 0.240 | 0.275 | 0.300 | 0.325 | 0.312 | 0.17 |
| Final Acid Number | 31.2 | 25.8 | 28.3 | 25.2 | 38.0 | 36.5 | 29.6 |
| Kinematic Viscosity of product, 100 °C , mm²/s | 13.3 | 13.7 | 25.2 | 23.2 | 13.2 | 12.2 | 23.8 |

| | | | | | | | |
|---|---|---|---|---|---|---|---|
| ^{∗}= run on a larger scale (pilot plant); rate not directly comparable | | | | | | | |

Ex. 28 through 31 (not covered by the present invention) illustrate the oxidation of slack wax with a different alkaline earth metal catalyst.

| Example: | Ref 28 | 29 | 30 | Ref 31 |
|---|---|---|---|---|
| Slack wax | 99.3 | 98.3 | 98.3 | 100 |
| Ca octoate (5% chemical in oil diluent) | | 1.68 | | |
| Ca neodecanoate (5% chemical in oil diluent) | | | 1.68 | |
| Mn octoate (12% chemical in oil diluent) | 0.7 | | | |
| Reaction Rate (AN/min) | 0.089 | 0.086 | 0.085 | 0.048 |
| Final Acid Number | 28.8 | 25.7 | 25.6 | 25.9 |
| Kinematic Viscosity of product, 100 °C , mm²/s | 5.44 | 5.34 | 5.07 | 5.42 |

The results show that calcium neodecanoate is effective as well as calcium octoate.

It is to be understood that the upper and lower amount, range, and ratio limits set forth herein may be independently combined. Similarly, the ranges and amounts for each element of the invention can be used together with ranges or amounts for any of the other elements.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of' and "consisting of," where "consisting of' excludes any element or step not specified and "consisting essentially of' permits the inclusion of additional un-recited elements or steps that do not materially affect the essential or basic and novel characteristics of the composition or method under consideration. The expression "consisting of' or "consisting essentially of," when applied to an element of a claim, is intended to restrict all species of the type represented by that element, notwithstanding the presence of "comprising" elsewhere in the claim.

While certain representative embodiments and details have been shown for the purpose of illustrating the subject invention, it will be apparent to those skilled in this art that various changes and modifications can be made therein without departing from the scope of the subject invention. In this regard, the scope of the invention is to be limited only by the following claims.

## Claims

1. A method for oxidizing an olefin wax of 10 to 100 carbon atoms, comprising
(a) mixing said olefin wax with a salt of an alkaline earth metal in an amount to provide 0.001 to 0.03 weight percent of the alkaline earth metal of the reaction mixture, the anion of said salt comprising 4 to 36 carbon atoms;
(b) heating said mixture to 100 to 180 °C, or 140-160 °C; and
(c) supplying to said heated mixture a molecular oxygen-containing gas; wherein the method is conducted in the substantial absence of manganese or cobalt compounds, such that the amount of manganese or cobalt is less than 1 % or less than 0.1% or less than 0.01 percent by weight of the amount of the alkaline earth metal from the alkaline earth metal salt.

2. The method of claim 1 wherein the olefin wax comprises an α-olefin of 16 to 35 or to 26 carbon atoms.

3. The method of claim 1 or claim 2 wherein the alkaline earth metal is calcium.

4. The method of any of claim 1 through 3 wherein the anion of said salt is a carboxylate.

5. The method of any of claims 1 through 4 wherein the salt of the alkaline earth metal comprises calcium di-2-ethylhexanoate (i.e., dioctoate).

6. The method of any of claims 1 through 5 wherein the amount of the salt of the alkaline earth metal is 0.01 to 0.25 weight percent, or about 0.08 weight percent, of the amount of the olefin wax.

7. The method of any of claims 1 through 6 wherein the oxygen-containing gas is air and is supplied at a pressure of 700 kPa to 1500 kPa.

8. The method of any of claims 1 through 7 wherein a portion of the alkaline earth metal salt is removed from the olefin wax after oxidation.

9. The method of any of claims 1 through 8 wherein the oxidized olefin or slack wax is reacted with an alcohol to provide ester functionality.

10. An oxidized olefin wax of 10 to 100 carbon atoms prepared by the method of any one of claims 1 through 9.

11. The oxidized olefin wax of claim 10 being an oxidized α-olefin.

12. A coating composition comprising the oxidized olefin wax of claim 10 or claim 11.

13. An article coated with the material of any one of claims 10 through 12.

## Patentansprüche

1. Verfahren zum Oxidieren eines Olefinwachses mit 10 bis 100 Kohlenstoffatomen, das Folgendes umfasst
(a) Mischen des Olefinwachses mit einem Salz eines Erdalkalimetalls in einer Menge, um 0,001 bis 0,03 Gew.-% des Erdalkalimetalls der Reaktionsmischung bereitzustellen, wobei das Anion des Salzes 4 bis 36 Kohlenstoffatome umfasst;
(b) Erhitzen der Mischung auf 100 bis 180 °C oder 140-160 °C; und
(c) Zuführen eines molekularen Sauerstoff enthaltenden Gases zu der erhitzten Mischung; wobei das Verfahren im Wesentlichen in Abwesenheit von Mangan- oder Kobaltverbindungen derart durchgeführt wird, dass die Menge von Mangan oder Kobalt weniger als 1 Gew.-% oder weniger als 0, 1 Gew.-% oder weniger als 0,01 Gew.-% der Menge des Erdalkalimetalls aus dem Erdalkalimetallsalz beträgt.

2. Verfahren nach Anspruch 1, wobei das Olefinwachs ein α-Olefin mit 16 bis 35 oder bis 26 Kohlenstoffatomen umfasst.

3. Verfahren nach Anspruch 1 oder 2, wobei das Erdalkalimetall Calcium ist.

4. Verfahren nach einem der Ansprüche 1 bis einschließlich 3, wobei das Anion des Salzes ein Carboxylat ist.

5. Verfahren nach einem der Ansprüche 1 bis einschließlich 4, wobei das Salz des Erdalkalimetalls Calcium-di-2-ethylhexanoat (d. h. Dioctoat) umfasst.

6. Verfahren nach einem der Ansprüche 1 bis einschließlich 5, wobei die Menge des Salzes des Erdalkalimetalls 0,01 bis 0,25 Gew.-% oder etwa 0,08 Gew.-% der Menge des Olefinwachses beträgt.

7. Verfahren nach einem der Ansprüche 1 bis einschließlich 6, wobei das Sauerstoff enthaltende Gas Luft ist und mit einem Druck von 700 kPa bis 1500 kPa zugeführt wird.

8. Verfahren nach einem der Ansprüche 1 bis einschließlich 7, wobei ein Abschnitt des Erdalkalimetallsalzes nach der Oxidation aus dem Olefinwachs entfernt wird.

9. Verfahren nach einem der Ansprüche 1 bis einschließlich 8, wobei das oxidierte Olefin oder Paraffinwachs mit einem Alkohol reagiert wird, um eine Esterfunktionalität bereitzustellen.

10. Oxidiertes Olefinwachs mit 10 bis 100 Kohlenstoffatomen, hergestellt durch das Verfahren nach einem der Ansprüche 1 bis einschließlich 9.

11. Oxidiertes Olefinwachs nach Anspruch 10, das ein oxidiertes α-Olefin ist.

12. Beschichtungszusammensetzung, die das oxidierte Olefinwachs nach Anspruch 10 oder 11 umfasst.

13. Erzeugnis, das mit dem Material nach einem der Ansprüche 10 bis einschließlich 12 beschichtet ist.

## Revendications

1. Procédé d'oxydation d'une cire d'oléfine de 10 à 100 atomes de carbone, comprenant
(a) le mélange de ladite cire d'oléfine avec un sel d'un métal alcalino-terreux en une certaine quantité pour fournir 0,001 à 0,03 pour cent en poids du métal alcalino-terreux du mélange réactionnel, l'anion dudit sel comprenant 4 à 36 atomes de carbone ;
(b) le chauffage dudit mélange à 100 à 180°C, ou à 140-160°C ; et
(c) la fourniture audit mélange chauffé d'un gaz contenant de l'oxygène moléculaire ; le procédé étant réalisé en l'absence substantielle de composés de manganèse ou de cobalt, de sorte que la quantité de manganèse ou de cobalt est inférieure à 1 % ou inférieure à 0,1 % ou inférieure à 0,01 % en poids de la quantité de métal alcalino-terreux provenant du sel de métal alcalino-terreux.

2. Procédé selon la revendication 1, dans lequel la cire d'oléfine comprend une α-oléfine de 16 à 35 ou à 26 atomes de carbone.

3. Procédé selon la revendication 1 ou la revendication 2, dans lequel le métal alcalino-terreux est le calcium.

4. Procédé selon l'une quelconque des revendications 1 à 3, dans lequel l'anion dudit sel est un carboxylate.

5. Procédé selon l'une quelconque des revendications 1 à 4, dans lequel le sel du métal alcalino-terreux comprend le di-2-éthylhexanoate de calcium (c'est-à-dire le dioctoate).

6. Procédé selon l'une quelconque des revendications 1 à 5, dans lequel la quantité de sel du métal alcalino-terreux est de 0,01 à 0,25 pour cent en poids, ou environ 0,08 pour cent en poids, de la quantité de cire d'oléfine.

7. Procédé selon l'une quelconque des revendications 1 à 6, dans lequel le gaz contenant de l'oxygène est de l'air et est fourni à une pression de 700 kPa à 1500 kPa.

8. Procédé selon l'une quelconque des revendications 1 à 7, dans lequel une partie du sel de métal alcalino-terreux est éliminée de la cire d'oléfine après oxydation.

9. Procédé selon l'une quelconque des revendications 1 à 8, dans lequel l'oléfine oxydée ou le gatsch est mis à réagir avec un alcool pour fournir une fonctionnalité ester.

10. Cire d'oléfine oxydée de 10 à 100 atomes de carbone préparée par selon procédé de l'une quelconque des revendications 1 à 9.

11. Cire d'oléfine oxydée selon la revendication 10, étant une α-oléfine oxydée.

12. Composition de revêtement comprenant la cire d'oléfine oxydée selon la revendication 10 ou la revendication 11.

13. Article revêtu du matériau selon l'une quelconque des revendications 10 à 12.
